# FASCICULE DE BREVET EUROPEEN

(11) **EP 2 262 440 B1**
(45) Date de publication et mention de la délivrance du brevet: **04.01.2012**
(21) Numéro de dépôt: 09742301.6
(22) Date de dépôt: 10.04.2009
(51) Int. Cl.: A61B 18/20

(54) **DISPOSITIF DE TRAITEMENT PAR EMISSION DE FLASHS LUMINEUX**
VORRICHTUNG FÜR BEHANDLUNGEN MIT BLITZLICHTEMISSIONEN
DEVICE FOR TREATING WITH FLASHLIGHT EMISSIONS

(30) Priorité: 10.04.2008 FR 0852427
(43) Date de publication de la demande: 22.12.2010
(73) Titulaire: Eurofeedback, 91017 Evry Cedex (FR)
(72) Inventeur: SAFRAOUI, Georges, F-91140 Villejust (FR)
(74) Mandataire: Tanty, François
(86) Numéro de dépôt international: PCT/FR2009/050650
(87) Numéro de publication internationale: WO 2009/136103

(56) Documents cités:
- EP-A- 0 726 083
- US-A- 4 444 190
- US-A- 5 001 616
- US-A1- 2002 173 780
- US-A1- 2006 217 586
- US-A1- 2006 271 028

## Description

La présente invention concerne les dispositifs de traitement par l'émission de flashs lumineux.

De tels dispositifs sont utilisés par exemple pour l'épilation et sont décrits notamment dans les demandes de brevet EP 1 602 340, EP 1 749 494 et le brevet européen EP 1 547 538 de la demanderesse.

D'une manière générale, la lumière émise par la lampe flash a un spectre large et elle doit être filtrée de manière à ce que le spectre de la lumière atteignant la peau soit adapté au traitement à effectuer.

Dans les machines connues de la demanderesse, un filtre absorbant rouge, par exemple de type SCHOTT RG610, est placé entre la lampe flash et un guide optique de la pièce à main conduisant la lumière vers la peau.

L'énergie lumineuse absorbée par le filtre est relativement importante en raison du filtrage propre sur la bande de blocage et au rendement inférieur à 100 % sur la bande passante. L'élévation de température du filtre peut conduire à son endommagement.

D'autres fabricants de dispositifs de traitement par émission de flashs lumineux utilisent non pas un filtre absorbant mais un filtre dichroïque.

L'avantage du filtre dichroïque est son moindre échauffement, ce qui le rend plus résistant.

L'efficacité d'un tel filtre n'est satisfaisante que sous incidence normale.

Dans le cas où la lumière incidente est produite par un laser, la lumière arrive perpendiculairement au filtre et ce dernier donne donc de bons résultats.

Par contre, la demanderesse a constaté que l'efficacité du filtre est médiocre pour filtrer la lumière émise par une lampe flash, car celle-ci atteint le filtre à des incidences non négligeables par rapport à la normale.

Par conséquent, la puissance émise par les dispositifs à lampe flash équipés d'un filtre dichroïque ne peut être aussi grande que souhaitable, sous peine de brûler la peau en raison du filtrage insuffisamment sélectif

EP 0 726 083 divulgue un dispositif comportant une lampe flash, un filtre interférentiel interchangeable et un filtre absorbant, placé en aval du filtre interférentiel.

Malgré la présence du filtre interférentiel, l'énergie reçue par le filtre absorbant reste relativement importante, ce qui limite la fluence incidence. Or, pour certaines applications, une fluence relativement élevée est souhaitable.

Il existe un besoin pour perfectionner encore les dispositifs de traitement par émission de flash lumineux comportant une lampe flash.

L'invention vise à répondre à ce besoin et elle y parvient en proposant un dispositif comportant, en aval de la lampe flash, un filtre dichroïque pour filtrer la lumière émise par la lampe flash et, en aval du filtre dichroïque, un filtre absorbant.

Le filtre dichroïque permet de réduire de manière significative l'énergie dissipée dans le filtre absorbant sans pour autant diminuer l'énergie utile pour réaliser le traitement recherché.

Le filtre dichroïque filtre la lumière atteignant le filtre absorbant et évite que celui-ci ne reçoive une énergie lumineuse excessive dans sa bande de blocage.

Le filtre dichroïque est formé sur le filtre absorbant, par exemple par une technique de dépôt sous vide sur le filtre absorbant, ce qui permet un refroidissement efficace à la fois du filtre dichroïque et du filtre absorbant.

Par ailleurs, l'emploi d'un filtre dichroïque insuffisamment sélectif ne constitue plus un inconvénient en raison de la présence du filtre absorbant et de la possibilité de refroidissement de ce dernier du fait de son accolement au filtre dichroïque.

Le filtre dichroïque peut notamment être au contact d'un liquide de refroidissement et le refroidissement du filtre absorbant peut s'effectuer à travers le filtre dichroïque d'une façon efficace.

L'invention permet d'augmenter si nécessaire l'énergie lumineuse émise par la lampe sans craindre un échauffement excessif du filtre absorbant, susceptible de l'endommager, ni entraîner un risque de brûlure de la peau.

Le guide optique peut être placé en aval du filtre absorbant, de manière à conduire la lumière sortant du filtre absorbant vers la peau.

Le filtre dichroïque est par exemple agencé, notamment pour l'épilation, de manière à éliminer les longueurs d'onde inférieures ou égales à 550 nm, mieux à 600 nm, et pour laisser passer les longueurs d'onde supérieures ou égales à 600 nm, mieux 610 nm.

Le filtre absorbant peut être agencé, notamment pour l'épilation, pour éliminer les longueurs d'onde inférieures ou égales à 550 nm, mieux à 600 nm, et pour laisser passer les longueurs d'onde supérieures ou égales à 600 nm, mieux 610 nm.

Pour d'autres applications, les longueurs d'onde peuvent être différentes.

La fluence de la lumière de la lampe flash en amont du filtre dichroïque est par exemple supérieure ou égale à 40 J/cm² (pour une impulsion de 20 ms par exemple), voire 100 J/cm² ou 150 J/cm².

La durée d'un flash de lumière est par exemple inférieure ou égale à 100 ms, mieux 50 ms, encore mieux 1 ms.

Le filtre dichroïque et le filtre absorbant, notamment lorsque le filtre dichroïque est formé sur le filtre absorbant, sont avantageusement refroidis par une circulation de liquide, par exemple le même liquide que celui servant à refroidir la lampe flash.

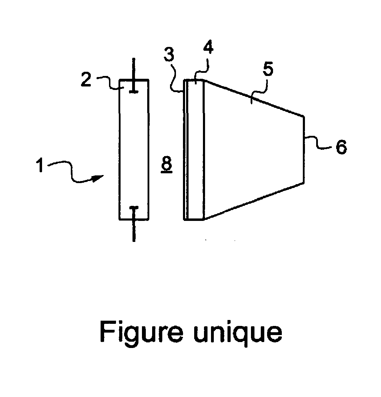

L'invention pourra être mieux comprise à la lecture de la description détaillée qui va suivre, d'un exemple non limitatif de mise en oeuvre de l'invention, et à l'examen du dessin annexé, sur lequel la figure 1 représente, de manière schématique, un exemple de dispositif réalisé conformément à l'invention.

Le dispositif de traitement par émission de flashs lumineux 1 représenté à la figure 1 de façon schématique comporte une lampe flash 2 qui est dans l'exemple considéré un tube à éclair rectiligne mais qui pourrait avoir une forme autre.

La lumière émise par la lampe flash 2 est filtrée par un filtre dichroïque 3 et par un filtre absorbant 4 avant d'atteindre un guide optique 5 qui conduit la lumière vers une fenêtre de sortie 6, cette dernière dirigeant la lumière vers la région à traiter.

La lampe flash 2 peut être refroidie par un liquide qui circule dans l'espace 8 entre la lampe 2 et le filtre dichroïque 3.

Le cas échéant, un verre transparent peut être disposé entre la lampe flash 2 et le filtre dichroïque 3.

Le liquide de refroidissement est par exemple de l'eau déminéralisée.

Dans l'exemple considéré, le filtre dichroïque 3 est formé directement sur une face du filtre absorbant 4 par une technique de dépôt sous vide, la nature des couches déposées ainsi que leurs épaisseurs étant choisies de manière à avoir la bande passante recherchée pour le filtre.

Le filtre dichroïque ne laisse par exemple passer que les longueurs d'ondes supérieures ou égales à 610 nm (λ_{c}=610 nm). La sélectivité du filtre absorbant est par exemple telle que celui-ci ne laisse passer également que les longueurs d'ondes supérieures ou égales à 610 nm (λ_{c}=610 nm).

La lampe flash peut émettre une lumière pulsée, par exemple à une cadence de plus d'un flash par seconde, la durée de chaque impulsion étant par exemple comprise entre 1 et 300 ms, une séquence d'impulsions comportant par exemple entre 1 et 10 éclairs sur la même région traitée.

La fluence de la lumière atteignant le filtre dichroïque est par exemple comprise entre 40 et 150 J/cm² (pour une impulsion de 20 ms par exemple).

La fluence de la lumière atteignant la peau est par exemple comprise entre 20 et 75 J/cm².

Bien entendu, l'invention n'est pas limitée à l'exemple qui vient d'être décrit.

Le filtre dichroïque et le filtre par absorption ont de préférence sensiblement les mêmes longueurs d'ondes de coupure λ_{c}, à ± 50 nm près, mieux à ± 20 nm près, mieux ± 10 nm près, de préférence ± 5 nm près.

Selon la puissance, le filtre absorbant peut être refroidi par air et non par liquide, grâce à la présence du filtre dichroïque. Un refroidissement par liquide à travers le filtre dichroïque est toutefois préférable.

L'expression « comportant un » doit être comprise comme étant synonyme de « comportant au moins un ».

## Revendications

1. Dispositif de traitement de la peau par émission de flash lumineux, comportant :
- une lampe flash, (2),
- un filtre dichroïque (3) pour filtrer la lumière émise par la lampe flash,
- un filtre absorbant (4) placé en aval du filtre dichroïque (3), **caractérisé par le fait que** le filtre dichroïque (3) est formé sur le filtre absorbant (4).

2. Dispositif selon la revendication 1, les filtres dichroïque et absorbant étant refroidis par une circulation d'un liquide, le filtre dichroïque étant au contact de ce liquide et le refroidissement du filtre absorbant s'effectuant à travers le filtre dichroïque.

3. Dispositif selon la revendication 1 ou 2, comportant un guide optique (5) en aval du filtre absorbant.

4. Dispositif selon l'une quelconque des revendications 1 à 3, le filtre dichroïque et le filtre absorbant ayant les mêmes longueurs d'onde de coupure à ± 50 nm près.

5. Dispositif selon l'une quelconque des revendications 1 à 4, le filtre dichroïque étant agencé pour laisser passer les longueurs d'onde supérieures ou égales à 600 nm, mieux 610 nm.

6. Dispositif selon lequel l'une quelconque des revendications précédentes, le filtre absorbant étant agencé pour laisser passer les longueurs d'onde supérieures ou égales à 600 nm, mieux 610 nm.

7. Dispositif selon l'une quelconque des revendications précédentes, la fluence en amont du filtre dichroïque étant supérieure ou égale à 40 J/cm².

8. Dispositif selon l'une quelconque des revendications précédentes, la durée d'une impulsion lumineuse étant inférieure ou égale à 100 ms.

9. Dispositif selon la revendication 2, le liquide refroidissant les filtres dichroïque et absorbant étant le même que celui refroidissant la lampe flash.

10. Dispositif selon la revendication 2 ou 9, le liquide de refroidissement étant de l'eau.

## Claims

1. A device for treating the skin by emitting flashes of light, the device comprising:
• a flash lamp (2);
• a dichroic filter (3) for filtering the light emitted by the flash lamp;
• an absorbent filter (4) placed downstream from the dichroic filter (3), **characterized in that** the dichroic filter (3) is formed on the absorbent filter (4).

2. A device according to claim 1, the dichroic and absorbent filters being cooled by circulating a liquid, the dichroic filter being in contact with the liquid and the absorbent filter being cooled through the dichroic filter.

3. A device according to claim 1 or 2, including a light guide (5) downstream from the absorbent filter.

4. A device according to any one of claims 1-3, the dichroic filter and the absorbent filter having the same cutoff wavelength to within ±50 nm.

5. A device according to any one of claims 1-4, the dichroic filter being arranged to pass wavelengths greater than or equal to 600 nm, better 610 nm.

6. A device according to any one of claims 1-5, the absorbent filter being arranged to pass wavelengths greater than or equal to 600 nm, better 610 nm.

7. A device according to any one of claims 1-6, the fluence upstream from the dichroic filter being greater than or equal to 40 J/cm².

8. A device according to any of claims 1-7, the duration of a light pulse being less than or equal to 100 ms.

9. A device according to claim 2, the liquid cooling the dichroic and absorbent filters being the same as the liquid cooling the flash lamp.

10. A device according to claim 2 or 9, the cooling liquid being water.

## Patentansprüche

1. Vorrichtung zur Behandlung der Haut durch Emission von Lichtblitzen, mit:
- einer Blitzlampe (2),
- einem dichroitischem Filter (3) zum Filtern des von der Blitzlampe emittierten Lichts,
- einem strahlabwärts des dichroitischen Filters (3) angeordneten Absorptionsfilter (4), **dadurch gekennzeichnet, dass** der dichroitische Filter (3) auf dem Absorptionsfilter (4) ausgebildet ist.

2. Vorrichtung nach Anspruch 1, bei der der dichroitische Filter und der Absorptionsfilter durch eine Zirkulation einer Flüssigkeit gekühlt werden, der dichroitische Filter mit dieser Flüssigkeit in Kontakt steht und die Kühlung des Absorptionsfilters durch den dichroitischen Filter hindurch erfolgt.

3. Vorrichtung nach Anspruch 1 oder 2, mit einem Lichtleiter (5) strahlabwärts des Absorptionsfilters.

4. Vorrichtung nach einem der Ansprüche 1 bis 3, bei der der dichroitische Filter und der Absorptionsfilter auf ± 50 nm genau die gleiche Cut-Off-Wellenlänge haben.

5. Vorrichtung nach einem der Ansprüche 1 bis 4, bei der der dichroitische Filter dazu ausgebildet ist, Wellenlängen von 600 nm oder mehr, vorzugsweise 610 nm oder mehr durchzulassen.

6. Vorrichtung nach einem der vorstehenden Ansprüche, bei der der Absorptionsfilter dazu ausgebildet ist, Wellenlängen von 600 nm oder mehr, vorzugsweise 610 nm oder mehr durchzulassen.

7. Vorrichtung nach einem der vorstehenden Ansprüche, bei der der Strahlungsfluss strahlabwärts des dichroitischen Filters größer oder gleich 40 J/cm² ist.

8. Vorrichtung nach einem der vorstehenden Ansprüche, bei der die Dauer eines Lichtblitzes kleiner oder gleich 100 ms ist.

9. Vorrichtung nach Anspruch 2, bei der die Flüssigkeit, die den dichroitischen Filter und den Absorpfionsfilter kühlt, die gleiche ist wie die Flüssigkeit, die die Blitzlampe kühlt.

10. Vorrichtung nach Anspruch 2 oder 9, bei der die Kühlflüssigkeit Wasser ist.
